(19) Europäisches Patentamt
European Patent Office
Office européen des brevets

(11) Veröffentlichungsnummer: **0 134 570**
**B1**

(12) # EUROPÄISCHE PATENTSCHRIFT

(45) Veröffentlichungstag der Patentschrift: **13.01.88**

(21) Anmeldenummer: **84110150.4**

(22) Anmeldetag: **25.08.84**

(51) Int. Cl.⁴: **C 07 C 121/00**, C 07 C 121/14, C 07 C 121/66, C 07 C 121/75, C 07 C 43/192, C 07 C 43/225, C 07 C 21/24, C 07 C 19/02, C 09 K 19/30, C 09 K 19/32, C 09 K 19/34

(54) **Anisotrope Verbindungen und Flüssigkristallmischungen.**

(30) Priorität: **10.09.83 DE 3332691**

(43) Veröffentlichungstag der Anmeldung: **20.03.85 Patentblatt 85/12**

(45) Bekanntmachung des Hinweises auf die Patenterteilung: **13.01.88 Patentblatt 88/2**

(84) Benannte Vertragsstaaten: **AT CH DE FR GB IT LI NL**

(56) Entgegenhaltungen:
**EP-A-0 084 194**
**DE-A-2 641 861**
**GB-A-2 092 146**
**GB-A-2 102 000**

(73) Patentinhaber: **Merck Patent Gesellschaft mit beschränkter Haftung, Frankfurter Strasse 250, D-6100 Darmstadt (DE)**

(72) Erfinder: **Huynh- Ba, Tuong, Dr., 47 Chemin des Osches, CH- 1009 Pully (CH)**
Erfinder: **Osman, Maged A. Dr., Lerchenrain 1, CH- 8046 Zürich (CH)**

**EP 0 134 570 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue anisotrope Verbindungen, die mindestens einen querpolarisierenden Substituenten tragen, und verminderte Viskositäten sowie erhöhte Klärpunkte bieten; die Erfindung betrifft ferner Flüssigkristallmischungen (FK-Mischungen), welche diese Verbindungen als Komponenten enthalten.

Bekanntlich werden für verschiedene Typen von elektrooptischen Anzeigen, z. B. für die sogenannten "Guest/Host-Displays" (GHD) und die homeotrop-nematischen Displays (HND), nematische FK-Mischungen mit negativen Werten von $\Delta\varepsilon$ benötigt, wobei $\Delta\varepsilon$ als DK-Anisotropie oder DKA bezeichnet wird und definiert ist als $\Delta\varepsilon = \varepsilon_{\parallel} - \varepsilon_{\perp}$, worin $\varepsilon_{\parallel}$ die Dielektrizitätskonstante (DK) parallel zur Moleküllängsachse und $\varepsilon_{\perp}$ die DK senkrecht zur Molekülachse bedeuten.

Ferner werden für bestimmte Betriebsarten der Anzeigen, z. B. Multiplexbetrieb, FK-Mischungen mit insgesamt positiver DKA, aber einem möglichst geringen Wert des Verhältnisses $\frac{\Delta\varepsilon}{\varepsilon_{\perp}}$ benötigt, d. h. Mischungen oder Verbindungen mit einem signifikanten Wert von $\varepsilon_{\perp}$.

Die Werte von $\varepsilon_{\parallel}$ bzw. $\varepsilon_{\perp}$ werden hauptsächlich vom Ausmass der Polarisierung des Moleküls in Richtung parallel bzw. quer zur Moleküllängsachse bestimmt; beispielsweise erhöht ein stark polarisierender Substituent, wie die Cyanogruppe oder ein Halogenatom, als Flügelgruppe – im folgenden auch als längspolarisierender Substituent bezeichnet - den Wert von $\varepsilon_{\parallel}$, während ein solcher Substituent in "lateraler", d. h. mehr oder weniger quer zur Moleküllängsrichtung stehender Position den Wert von $\varepsilon_{\perp}$ erhöht; diese lateralen Substituenten werden hier auch als "querpolarisierende" Substituenten bezeichnet.

Die FK-Mischungen bzw. deren Komponenten müssen aber auch eine Reihe von weiteren Eigenschaften erfüllen, wozu insbesondere eine hohe Stabilität in fotochemischer, allgemein chemischer, thermischer und elektrochemischer Hinsicht sowie eine möglichst geringe Viskosität, geringe optische Anisotropie (geringe Doppelbrechungsneigung) und möglichst hohe Klärpunkte gehören.

Aus DE-A 26 41 861 waren anisotrope α-Cyanstilbenverbindungen mit negativer dielektrischer Anisotropie bekannt, worin eine Cyanogruppe in einem Vinylen-Brückenglied als querpolarisierender Substituent fungiert. Diese Materialien sind jedoch infolge ihrer geringen Stabilität in kommerziellen FK-Mischungen nicht einsetzbar.

Gemäss Stand der Technik werden negative DKA-Werte bzw. höhere $\varepsilon_{\perp}$-Beiträge allgemein dadurch erzielt, dass man einen aromatischen cyclischen Rest, meist einen Benzolring, mit mindestens einem querpolarisierenden Substituenten versieht.

Die bekannten Verbindungen mit negativer DKA besitzen daher als charakteristisches Element Kerne mit der Struktur

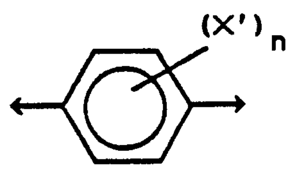

in der X' die querpolarisierende Gruppe, z. B. Cyano oder Halogen ist, n 1 oder 2 bedeutet und die Pfeile annähernd der Moleküllängsachse entsprechen. Solche Verbindungen sind z. B. in den DE-A-2 240 864, 2 613 293, 2 835 662, 2 836 086, 2 853 728, 2 937 770 und EP-A-0 023 728 beschrieben. Aus der europäischen Patentanmeldung Nr. 79200259.4 (EP-A-0 019 665) ist es ferner bekannt, mindestens einen querpolarisierenden Substituenten mit einem längspolarisierenden Substituenten zu kombinieren, um anisotrope Verbindungen mit positiver DKA und kleinen Werten des Verhältnisses $\frac{\Delta\varepsilon}{\varepsilon_{\perp}}$ zu gewinnen.

Gemeinsam ist den bekannten Verbindungen eine durch den oder die querpolarisierenden Substituenten bedingte Verbreiterung des Moleküls, die nachteilig ist, weil sie zu einer Erniedrigung des Klärpunktes führt und häufig eine relativ hohe Viskosität bedingt; da die lateralen polarisierenden Gruppen praktisch nur in aromatische Reste eingeführt werden können, stellt die Notwendigkeit der Anwesenheit eines aromatischen Restes eine weitere Beschränkung dar.

Der Erfindung liegt die Aufgabe zugrunde, neue anisotrope Verbindungen anzugeben, die mindestens einen querpolarisierenden Substituenten tragen, aber keine an cyclischen Resten hängenden querpolarisierende Gruppen aufweisen, so dass die obigen Nachteile bzw. Beschränkungen vermieden werden können.

Die Aufgabe wird erfindungsgemäss durch neue anisotrope Verbindungen der Formel (1) gelöst, die mindestens zwei cyclische Reste besitzen, welche durch ein bestimmtes, die polarisierende Gruppe tragendes Brückenglied (im folgenden auch als Hauptbrücke bezeichnet) miteinander verbunden sind:

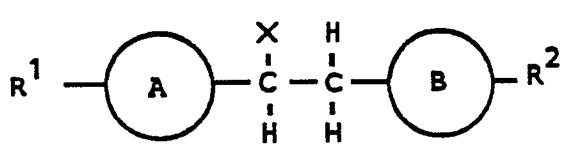

(1)

Der Ring A ist ein cycloaliphatischer Ring der Formeln (1a) oder (1b)

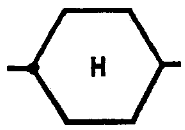

(1a)

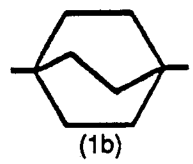

(1b)

d. h. ein trans-1,4-Cyclohexylrest oder ein 1,4-Bicyclo-(2,2,2)-octylrest.

Der Ring B kann ebenfalls ein cycloaliphatischer Ring der Formeln (1a), (1b) oder ein aromatischer Rest der Formeln (1c) oder (1d) sein:

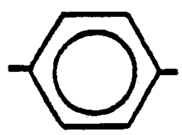

(1c)

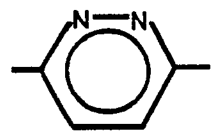

(1d)

d. h. ein 1,4-Phenylrest oder ein 3,6-Pyridazinylrest.

Die polarisierende Gruppe X, die ein Halogenatom oder die Cyanogruppe ist, muss an demjenigen C-Atom der Hauptbrücke hängen, welches mit dem bzw. einem cycloaliphatischen Rest direkt verbunden ist. Da der Ring A stets cycloaliphatisch sein muss, der Ring B hingegen cycloaliphatisch oder aromatisch sein kann, ergibt dies die in Formel (1) dargestellte Position von X; wenn der Ring B ebenfalls ein cycloaliphatischer Rest ist, könnte X auch an dem C-Atom der Hauptbrücke hängen, das direkt mit dem Ring B verbunden ist.

Die Gruppen $R^1$, $R^2$ in Formel (1) sind gleiche oder verschiedene Flügelgruppen der Formel (1e)

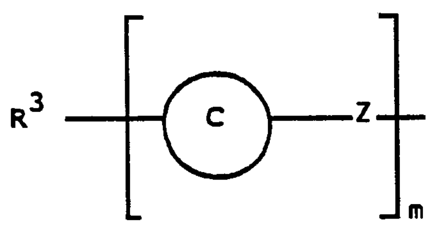

(1e)

in der der Ring C die für Ring B angegebene Bedeutung hat, d. h. einen Rest der Formeln (1a), (1b), (1c) oder (1d) darstellt und somit ebenfalls cycloaliphatisch oder aromatisch sein kann; Ring C in Formel (1e) ist aber fakultativ, d. h. m ist 0 oder 1.

Die Brücke Z in Formel (1e) ist die Kovalenzbindung oder eine Nebenbrückengruppe der Formeln $-CH_2O-$, $-C(X^1)(H)-CH_2-$ oder -COO-, wobei $X^1$ Wasserstoff ist oder die für X angegebene Bedeutung hat, und zwar jeweils auch in umgekehrter Folge, wobei aber die nachfolgend erläuterten Massgaben (a) und (b) zu berücksichtigen sind.

Wenn $X^1$ in der Nebenbrücke die für X genannte Bedeutung hat, kann eine zusätzliche laterale Polarisierung bewirkt werden. Für viele Zwecke wird Z in Form der Kovalenzbindung bevorzugt.

$R^3$ kann Wasserstoff darstellen, sofern m 1 ist und der Ring C ein cyclischer Rest der Formeln (1c) oder (1d) ist, oder allgemein Alkyl- ($H_{2n+1}C_n-$), Alkoxy- ($H_{2n+1}C_n-O-$), Alkoxycarbonyl- ($H_{2n+1}C_n-OC(O)-$) oder Alkylcarbonyloxygruppen ($H_{2n+1}C_n-C(O)-O-$) bedeuten, deren Alkylteil 1-12 C-Atome (n = 1-12) in gerader oder verzweigter und gegebenenfalls chiraler Kette enthält. Beispiele für die Alkylteile der genannten Gruppen sind daher Methyl-, Ethyl-, Propyl-, Butyl-, Pentyl-, Hexyl-, Heptyl-, Octyl-, Nonyl-, Decyl-, Undecyl- und Dodecylgruppen, einschliesslich der isomeren bzw. chiralisomeren Alkyle, wie 2-Methylbutyl, 2-Methylbutoxy, 2-Methylpentyl, 3-Methylheptyl usw. Diese Alkylteile sind vorzugsweise geradkettig und haben 3 - 7 C-Atome.

Die Alkylteile von $R^3$ können einen oder mehrere Substituenten, und zwar Halogenatome oder Cyanogruppen tragen, wobei aber an jeweils einem C-Atom des Alkylteils höchstens jeweils ein derartiger Substituent hängt. Vorzugsweise enthalten die Alkylteile von $R^3$ ein oder höchstens zwei derartige Substituenten, und zwar vorzugsweise an solchen C-Atomen des Alkylteils, die vom zugehörigen Ring nicht allzuweit entfernt sind, z. B. an 1-, 2- oder 3-ständigen Atomen der Flügelgruppenkette hängen, wenn als 1-ständiges Atom der Kette das an den zugehörigen Ring gebundene Atom (C oder O) bezeichnet wird. Dabei kann der Beitrag zur Längs- bzw. Querpolarisierung unter Umständen, insbesondere wenn der Substituent Cyano ist, dadurch erhöht werden, dass der Substituent an einem geradzahligen bzw. ungeradzahligen Atom der Kette der Flügelgruppe hängt.

Weiter werden als Substituenten des Alkylteils von $R^3$ meist Fluor, Chlor oder Cyano bevorzugt.

Die tatsächliche Konformation der Hauptbrücke von Formel (1) entspricht dem Schema

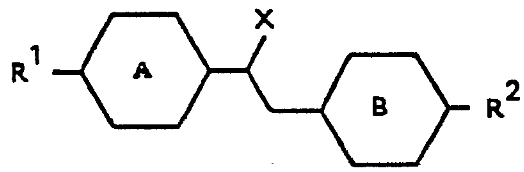

und zeigt, dass die durch den brückenständigen lateral polarisierenden Rest X bedingte Molekülverbreiterung bei den erfindungsgemässen anisotropen Verbindungen geringer ist, als bei den bekannten anisotropen Verbindungen mit ringständigem X.

Konkrete Vergleichswerte für die überraschenden Eigenschaftsverbesserungen von erfindungsgemässen Verbindungen (1) mit brückenständigen Lateralpolarisierungsgruppen gegenüber vergleichbaren (bezüglich DKA) Verbindungen mit ringständigen Lateralpolarisierungsgruppen werden weiter unten in Beispiel 2 gegeben.

Es ist ferner überraschend, dass mit brückenständigen und stark polarisierend wirkenden Gruppen überhaupt Verbindungen mit der für FK-Mischungen erforderlichen Stabilität erhältlich sind. Bekannte Verbindungen mit brückenständigen Cyanogruppen erwiesen sich fast immer als instabil und die Untersuchungen der Anmelderin zeigten, dass z. B. Verbindungen mit "Cyanobenzylstruktur"

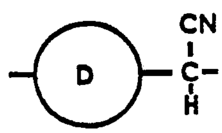

in der D ein aromatischer Ring ist, ebenso wie Verbindungen mit "Benzylätherstruktur"

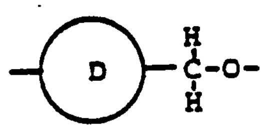

hier keine Verbindungen mit ausreichender Stabilität ergeben.

Für erfindungsgemässe Verbindungen der Formel (1) gelten daher folgende Massgaben oder Einschränkungen:

(a) an keinen aromatischen Rest der Formeln (1c) oder (1d) sind Gruppen der Formeln

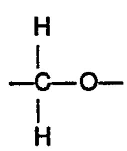

und

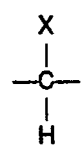

direkt mit den C-Atomen dieser Gruppen gebunden ausser wenn X = F, und

(b) $R^3$ in Formel (1e) ist nicht Wasserstoff, wenn m 0 bedeutet.

Bevorzugte Gruppen erfindungsgemässer Verbindungen der Formel (1) haben folgende Merkmale, einzeln oder in Kombination:

- X bedeutet die Cyanogruppe;
- wenn X Halogen ist, wird Fluor und Chlor bevorzugt;
- wenn $R^3$ einen substituierten Alkylteil enthält, trägt dieser vorzugsweise einen oder zwei Substituenten, wobei als Halogenatome Fluor und Chlor bevorzugt sind;
- das Molekül der Formel (1) enthält vorzugsweise insgesamt höchstens eine Carboxylgruppe;
- das Molekül der Formel (1) enthält vorzugsweise höchstens einen aromatischen Ring der Formeln (1c), (1d);
- das Molekül der Formel (1) enthält vorzugsweise insgesamt höchstens drei cyclische Reste;

- das Molekül der Formel (1) enthält zwei oder drei cycloaliphatische Reste der Formeln (1a), (1b);
- das Molekül der Formel (1) enthält zwei Flügelgruppen mit Alkylteilen, von denen jeder 3-9 C-Atome umfasst;
- der oder die (höchstens zwei) Substituenten der Alkylteile von $R^3$ hängen an solchen C-Atomen der Alkylkette, welche vom zugehörigen, d. h. nächsten Ring durch nicht mehr als 2 Atome getrennt sind.

Ebenfalls bevorzugt werden für viele Verwendungszwecke Verbindungen der folgenden Formeln 2-5:

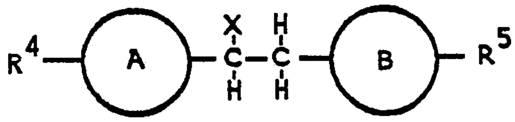 (2)

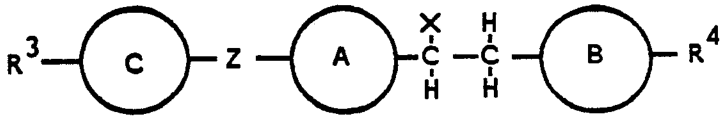 (3)

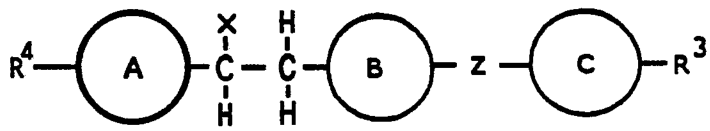 (4)

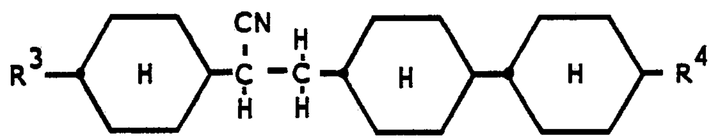 (5)

in welchen A, B, C, X, Z und $R^3$ die oben angegebene Bedeutung haben und $R^4$, $R^5$ die für $R^3$ angegebene Bedeutung mit Ausnahme von Wasserstoff haben; die Erfindung ist aber nicht auf zwei- oder dreikernige Verbindungen (1) beschränkt, da sowohl $R^1$ und $R^2$ gleiche oder verschiedene Gruppen der Formel (1e) bedeuten können.

Erfindungsgemäß sind ferner Flüssigkristallmischungen, die mindestens eine Verbindung der Formel (1) enthalten, z. B. in Anteilen von 1-30 Gew.-%, wobei die Mischung auch mehrere verschiedene Verbindungen der Formel (1), z. B. in einem Anteil von insgesamt 5-70 Gew.-%, insbesondere 15-65 Gew.-% enthalten kann. Als weitere Komponenten können die erfindungsgemäßen Fk-Mischungen bekannte anisotrope Verbindungen sowie die dem Verwendungszweck entsprechenden Zusätze, wie Farbstoffe, insbesondere pleochroitische Farbstoffe, optisch aktive bzw. cholesterische Komponenten und dergleichen enthalten.

Die neuen Verbindungen der Formel (1) sind nach verschiedenen an sich bekannten Verfahren erhältlich; die Verbindungen (1), bei welchen X in der Hauptbrücke die Cyanogruppe bedeutet, können z. B. direkt durch Kondensation nach folgendem Schema I erhalten werden:

**Schema I**

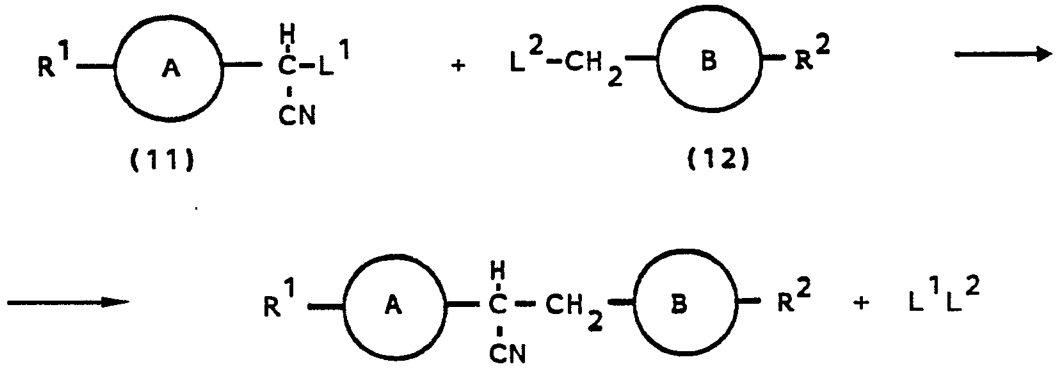

(11) (12)

(1)

wobei $R^1$, $R^2$, A und B die oben angegebene Bedeutung haben und $L^1$, $L^2$ Abgangsgruppen sind, wie z. B. $L^1$=H, $L^2$=Br usw. Zweckmässigerweise wird dabei in einem flüssigen Medium und bei erhöhter Temperatur in Gegenwart von Substanzen kondensiert, welche die Kondensation beschleunigen, wie z. B. metallisches Natrium, oder die als Nebenprodukt entstehende Verbindung $L^1$ $L^2$ binden.

Geeignete Ausgangsverbindungen der Formeln (11) und (12) sind entweder bekannt oder nach folgendem Schema II erhältlich:

**Schema II**

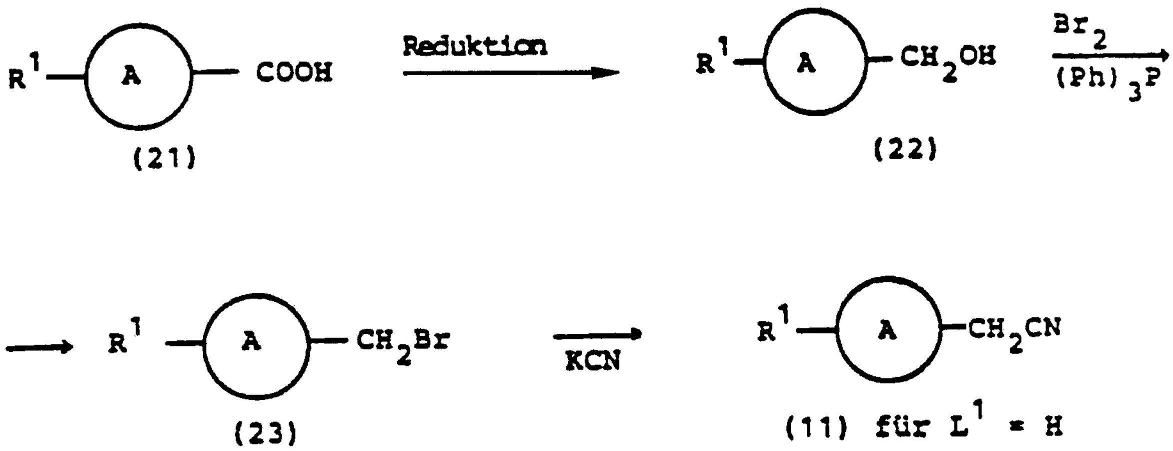


(21) (22) (23) (11) für $L^1$ = H

Geeignete Ausgangsverbindungen (21) für die Synthese nach Schema II sind bekannt oder können ähnlich wie die bekannten Verbindungen erhalten werden.

Die Reduktion kann in an sich bekannter Weise, z. B. mit Lithiumaluminiumhydrid erfolgen; die Bromierung erfolgt meist zweckmässig mit elementarem Brom in Gegenwart von Triphenylphosphin und die Umsetzung mit KCN lässt sich nach Org. Synth. Coll. Vo. 3, 852 (1955) erzielen.

In analoger Weise können nach Schema II auch die den Verbindungen (23) entsprechenden Ausgangsverbindungen (12)

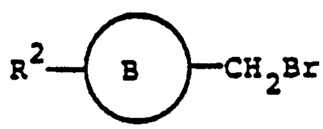

(12) für $L^2$ = Br

für die Synthese nach Schema I erhalten werden, bei welchen B einen aromatischen Rest der Formeln (1c) oder (1d) bedeutet und $R^1$=$R^2$ ist, indem man anstelle der Verbindungen (21) die entsprechenden Carbonsäuren der Formel (210)

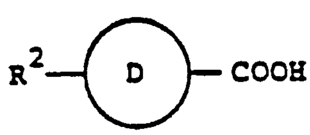

(210)

verwendet, in welcher der Ring D einen Rest der Formeln (1c) oder (1d) bedeutet.

Allgemeine Beispiele für geeignete Verbindungen der Formeln (21) bzw. (210) sind die folgenden:

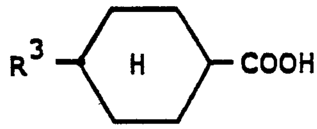

(21/1)

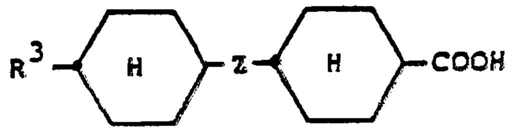

(21/2)

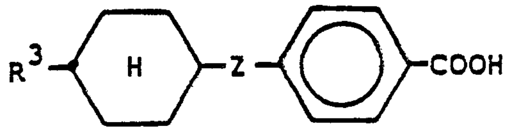

(210/1)

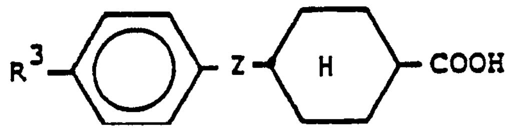

(21/3)

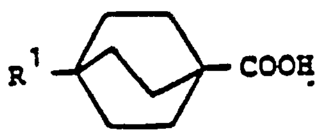

(21/4)

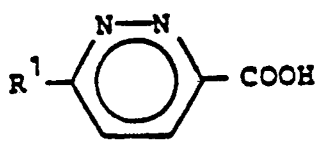

(210/2)

in welchen $R^1$ und $R^3$ die oben angegebene Bedeutung haben. Die Herstellung von Säuren der Formel (21/4) ist beispielsweise in Mol. Cryst. Liqu. Cryst. 75 (1981) 95 beschrieben. Die Säuren der Formel (210/2) können aus den in Z. für Chemie, 17 (1977) 333 beschriebenen Chlor- oder Brompyridazinderivaten mit $CO_2$/Mg (Grignard) erhalten werden.

Die Verbindungen der Formel (1), bei welchen X in der Hauptbrücke Halogen bedeutet, sind nach folgendem Schema III erhältlich:

**Schema III**

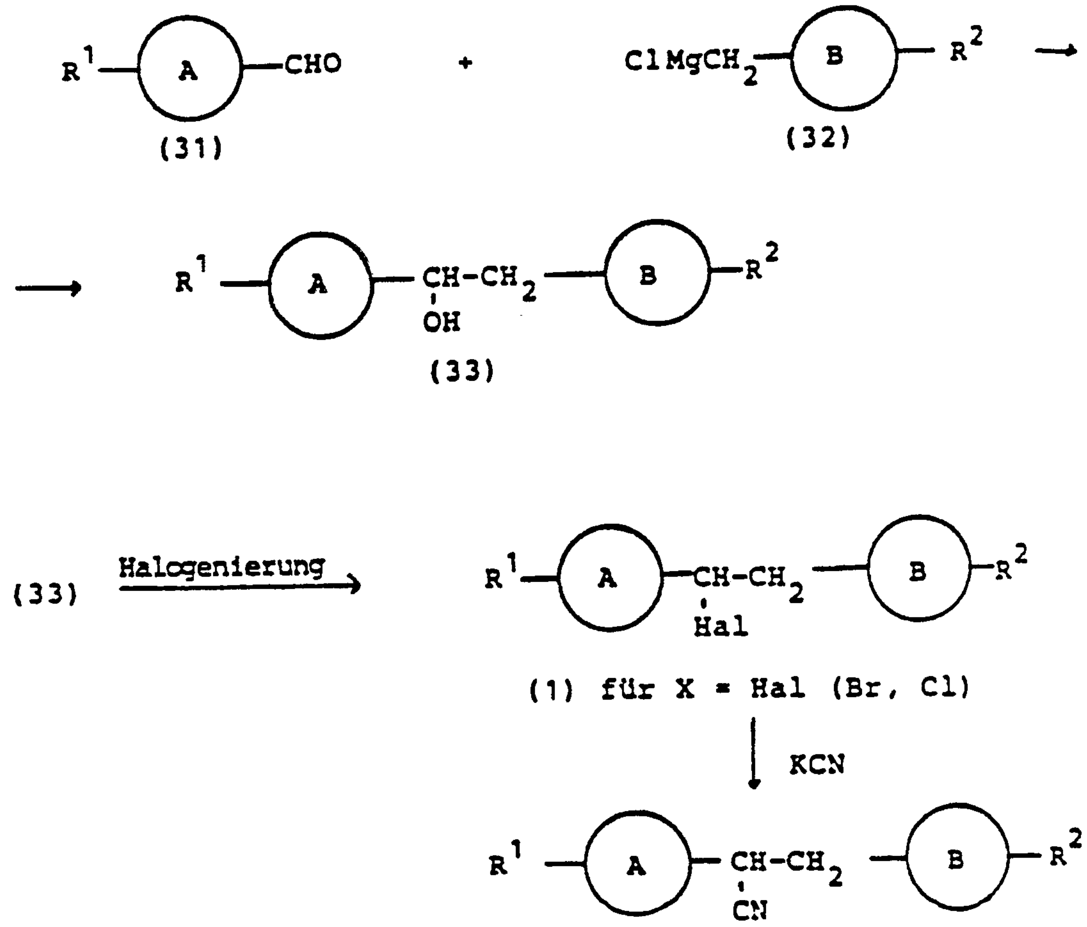


(31) (32) (33)

(1) für X = Hal (Br, Cl)

(1) für X = CN

Die Umsetzung von (31) mit (32) ist eine an sich bekannte Grignard-Synthese und die Halogenierung von (33) kann mit Thionylchlorid bzw. einem Phosphorhalogenid erzielt werden.

Die entsprechenden Verbindungen (1) für Hal=F oder I sind durch an sich bekannte Transhalogenierungsmethoden, z. B. nach Finkelstein, aus den Produkten der Synthese nach Schema III erhältlich. Zur Einführung von Fluor durch Transhalogenierung sind meist auch die von Gerstenberger et al in Angew. Chemie 93 (1981) 659 beschriebenen Methoden geeignet.

Wiederum sind geeignete Ausgangsverbindungen der Formeln (31) und (32) entweder bekannt oder können ähnlich wie die bekannten Verbindungen erhalten werden.

Ein weiteres Verfahren zur Herstellung von Verbindungen der Formel (1) besteht darin, dass man die nach Schema I oder III erhaltenen Verbindungen (1), z. B. an den Flügelgruppen $R^1$, $R^2$ modifiziert, oder indem man die Verbindungen, bei welchen X in der Hauptbrücke Halogen, wie Brom ist, mit Kaliumcyanid behandelt.

Weiterhin können anstelle der in Schema I und III angegebenen Ausgangsverbindungen der Formeln (11), (12), (31) und (32) die entsprechenden Vorverbindungen verwendet werden, in welchen anstelle der Flügelgruppen $R^1$, $R^2$ geeignete reaktive Gruppen, z. B. Halogenatome, Hydroxylgruppen, Carboxylgruppen oder dergleichen, stehen und diese dann nach Beendigung der Synthesewege von I bzw. III nach an sich bekannten Methoden in die gewünschten Gruppen $R^1$, $R^2$ umgewandelt werden.

Der weiteren Erläuterung der Erfindung dienen die folgenden Beispiele.

**Beispiel 1**

Herstellung von 1,2-Bis-(4-trans-pentylcyclohexyl)-1-cyanoethan (Formel (2), $R^4=R^5$=n-Pentyl, X=CN, A=B=(1a))

In einer Lösung von 1,93 g (0,01 Mol) 4-trans-Cyanomethyl-1-pentylcyclohexan in 10 ml Benzol wurden 0,5 g metallisches Natrium suspendiert. Die Suspension wurde 60 min auf Rückflusstemperatur erhitzt. Dann wurden 2,02 g (0,01 Mol) 4-trans-Bromomethyl-1-pentylcyclohexan unter Rühren zugetropft.

Das Reaktionsgemisch wurde nach Beendigung der Umsetzung auf verdünnte kalte Salzsäure gegossen und die entstandene Mischung mit Dimethyläther extrahiert. Das rohe Zielprodukt wurde durch Chromatographie über Kieselgel gereinigt, Schmelzpunkt 41,3°C, Klärpunkt 61,5°C.

**Beispiel 2**

Herstellung von 1-(4'-trans-Pentyl-4-trans-bi-cyclohexyl)-2-(4-trans-pentylcyclohexyl)-2-cyanoethan (Formel (5), $R^3=R^4$=n-Pentyl)

1,93 g (0,01 Mol) 4-trans-Cyanomethyl-1-pentylcyclohexan in 10 ml Benzol wurden mit 0,5 g Natrium versetzt und 60 min unter kräftigem Rühren auf Rückflusstemperatur erhitzt.

Die so entstandene Dispersion wurde unter Rühren mit 0,01 Mol 4-trans-Bromomethyl-1-(4-trans-pentylcyclohexyl)-cyclohexan versetzt und bis zur Beendigung der Umsetzung erhitzt. Das Reaktionsgemisch wurde auf HCl/Eis-Mischung gegossen. Aus der erhaltenen Mischung wurde das Rohprodukt mit Dimethyläther extrahiert und zur Reinigung über Kieselgel chromatographiert, Schmelzpunkt 71,5°C, Klärpunkt 173°C.

Zum Vergleich der erfindungsgemässen Zielverbindung dieses Beispiels wurde die nicht-erfindungsgemässe Verbindung der Formel (V)

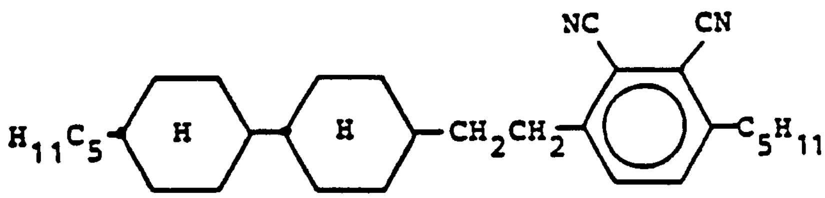

herangezogen, weil diese eine annähernd gleich negative DKA wie die Verbindung von Beispiel 2 zeigt, obwohl sie hierfür zwei ringständige Cyanogruppen benötigt. Dieser Vergleich zeigt die überraschenden Vorteile von erfindungsgemässen Verbindungen (1) mit brückenständiger, lateral polarisierender Gruppe gegenüber dem Stand der Technik in doppelter Hinsicht: zum einen kann erfindungsgemäss bereits mit einer einzigen, aber brückenständigen Cyanogruppe eine praktisch ebenso negative DKA erzielt werden, wie mit zwei ringständigen lateralen Cyanogruppen; dies ist wahrscheinlich darauf zurückzuführen, dass die brückenständige Cyanogruppe praktisch kein Polarisierungsmoment in Richtung der Moleküllängsachse erzeugt, während die ringständigen Cyanogruppen erhebliche, sich aber gegenseitig aufhebende Polarisierungsmomente in dieser Richtung bewirken.

Zum anderen hat die erfindungsgemässe Verbindung eine sehr breite Mesophase (71,5 bis 173°C), während die Vergleichsverbindung bei 163°C schmilzt und überhaupt keine Mesophase besitzt.

Analog werden hergestellt:

1-(4'-trans-Propyl-4-trans-bicyclohexyl)-2-(4-trans-propylcyclohexyl)-2-cyanoethan
1-(4'-trans-Propyl-4-trans-bicyclohexyl)-2-(4-trans-butylcyclohexyl)-2-cyanoethan
1-(4'-trans-Propyl-4-trans-bicyclohexyl)-2-(4-trans-pentylcyclohexyl)-2-cyanoethan
1-(4'-trans-Propyl-4-trans-bicyclohexyl)-2-(4-trans-hexylcyclohexyl)-2-cyanoethan
1-(4'-trans-Propyl-4-trans-bicyclohexyl)-2-(4-trans-heptylcyclohexyl)-2-cyanoethan
1-(4'-trans-Butyl-4-trans-bicyclohexyl)-2-(4-trans-propylcyclohexyl)-2-cyanoethan
1-(4'-trans-Butyl-4-trans-bicyclohexyl)-2-(4-trans-butylcyclohexyl)-2-cyanoethan
1-(4'-trans-Butyl-4-trans-bicyclohexyl)-2-(4-trans-pentylcyclohexyl)-2-cyanoethan
1-(4'-trans-Butyl-4-trans-bicyclohexyl)-2-(4-trans-hexylcyclohexyl)-2-cyanoethan
1-(4'-trans-Butyl-4-trans-bicyclohexyl)-2-(4-trans-heptylcyclohexyl)-2-cyanoethan
1-(4'-trans-Pentyl-4-trans-bicyclohexyl)-2-(4-trans-propylcyclohexyl)-2-cyanoethan
1-(4'-trans-Pentyl-4-trans-bicyclohexyl)-2-(4-trans-butylcyclohexyl)-2-cyanoethan
1-(4'-trans-Pentyl-4-trans-bicyclohexyl)-2-(4-trans-hexylcyclohexyl)-2-cyanoethan
1-(4'-trans-Pentyl-4-trans-bicyclohexyl)-2-(4-trans-heptylcyclohexyl)-2-cyanoethan
1-(4'-trans-Heptyl-4-trans-bicyclohexyl)-2-(4-trans-propylcyclohexyl)-2-cyanoethan
1-(4'-trans-Heptyl-4-trans-bicyclohexyl)-2-(4-trans-butylcyclohexyl)-2-cyanoethan
1-(4'-trans-Heptyl-4-trans-bicyclohexyl)-2-(4-trans-pentylcyclohexyl)-2-cyanoethan
1-(4'-trans-Heptyl-4-trans-bicyclohexyl)-2-(4-trans-hexylcyclohexyl)-2-cyanoethan
1-(4'-trans-Heptyl-4-trans-bicyclohexyl)-2-(4-trans-heptylcyclohexyl)-2-cyanoethan
1-(4-trans-Hexylcyclohexyl)-2-(4'-trans-propyl-4-trans-bicyclohexyl)-2-cyanoethan

1-(4-trans-Hexylcyclohexyl)-2-(4'-trans-butyl-4-trans-bicyclohexyl)-2-cyanoethan
1-(4-trans-Hexylcyclohexyl)-2-(4'-trans-pentyl-4-trans-bicyclohexyl)-2-cyanoethan
1-(4-trans-Hexylcyclohexyl)-2-(4'-trans-hexyl-4-trans-bicyclohexyl)-2-cyanoethan
1-(4-trans-Hexylcyclohexyl)-2-(4'-trans-heptyl-4-trans-bicyclohexyl)-2-cyanoethan
1-(4-trans-Propylcyclohexyl)-2-(4'-trans-propyl-4-trans-bicyclohexyl)-2-cyanoethan
1-(4-trans-Propylcyclohexyl)-2-(4'-trans-butyl-4-trans-bicyclohexyl)-2-cyanoethan
1-(4-trans-Propylcyclohexyl)-2-(4'-trans-pentyl-4-trans-bicyclohexyl)-2-cyanoethan
1-(4-trans-Propylcyclohexyl)-2-(4'-trans-hexyl-4-trans-bicyclohexyl)-2-cyanoethan
1-(4-trans-Propylcyclohexyl)-2-(4'-trans-heptyl-4-trans-bicyclohexyl)-2-cyanoethan

**Beispiel 3**

Herstellung von 1,2-Bis-(4-trans-propylcyclohexyl)-1-cyanoethan (Formel (2), $R^4$=$R^5$=n-Propyl, X=CN, A=B=(1a))

Nach dem in Beispiel 1 beschriebenen Verfahren wurde das Zielprodukt durch Umsetzung von 4-trans-Cyanomethyl-1-propylcyclohexan und 4-trans-Bromomethyl-1-propylcyclohexan gewonnen, Schmelzpunkt 45,7°C, Klärpunkt (37,0)°C.

Weitere Beispiele für erfindungsgemässe Verbindungen sind im folgenden genannt:

1,2-Bis-(4-trans-propylcyclohexyl)-1-fluoro-ethan
1,2-Bis-(4-trans-propylcyclohexyl)-1-chloro-ethan
1,2-Bis-(4-trans-propylcyclohexyl)-1-bromo-ethan
1,2-Bis-(4-trans-propylcyclohexyl)-1-cyano-ethan
1,2-Bis-(4-trans-fluoropropylcyclohexyl)-1-cyano-ethan

1,2-Bis-(4-trans-propyloxycarbonylcyclohexyl)-1-fluoro-ethan
1,2-Bis-(4-trans-propyloxycarbonylcyclohexyl)-1-chloro-ethan
1,2-Bis-(4-trans-propyloxycarbonylcyclohexyl)-1-bromo-ethan
1,2-Bis-(4-trans-propyloxycarbonylcyclohexyl)-1-cyano-ethan
1,2-Bis-(4-trans-fluoropropyloxycarbonylcyclohexyl)-1-cyanoethan

1-(4'-trans-Propyl-4-trans-bicyclohexyl)-2-(4-trans-propylcyclohexyl)-2-fluoro-ethan
1-(4'-trans-Propyl-4-trans-bicyclohexyl)-2-(4-trans-propylcyclohexyl)-2-chloro-ethan
1-(4'-trans-Propyl-4-trans-bicyclohexyl)-2-(4-trans-propylcyclohexyl)-2-bromo-ethan
1-(4'-trans-Propyl-4-trans-bicyclohexyl)-2-(4-trans-propyloxycarbonylcyclohexyl)-2-fluoro-ethan
1-(4'-trans-Propyl-4-trans-bicyclohexyl)-2-(4-trans-propyloxycarbonylcyclohexyl)-2-chloro-ethan
1-(4'-trans-Propyl-4-trans-bicyclohexyl)-2-(4-trans-propyloxycarbonylcyclohexyl)-2-bromo-ethan
1-(4'-trans-Propyl-4-trans-bicyclohexyl)-2-(4-trans-propyloxycarbonylcyclohexyl)-2-cyano-ethan

1-(4-trans-(2-(4-trans-Propylcyclohexyl)-ethyl)-cyclohexyl)-2-(4-trans-propylcyclohexyl)-2-fluoro-ethan
1-(4-trans-(2-(4-trans-Propylcyclohexyl)-ethyl)-cyclohexyl)-2-(4-trans-propylcyclohexyl)-2-chloro-ethan
1-(4-trans-(2-(4-trans-Propylcyclohexyl)-ethyl)-cyclohexyl)-2-(4-trans-propylcyclohexyl)-2-bromo-ethan
1-(4-trans-(2-(4-trans-Propylcyclohexyl)-ethyl)-cyclohexyl)-2-(4-trans-propylcyclohexyl)-2-cyano-ethan

1-(4-trans-(2-(4-trans-Propylcyclohexyl)-1-fluoroethyl)-cyclohexyl)-2-(4-trans-propylcyclohexyl)-2-fluoro-ethan,
1-(4-trans-(2-(4-trans-Propylcyclohexyl)-1-fluoroethyl)-cyclohexyl)-2-(4-trans-propylcyclohexyl)-2-cyano-ethan,
1-(4-trans-(2-(4-trans-Propylcyclohexyl)-1-cyanoethyl)-cyclohexyl)-2-(4-trans-propylcyclohexyl)-2-fluoro-ethan,
1-(4-trans-(2-(4-trans-Propylcyclohexyl)-1-cyanoethyl)-cyclohexyl)-2-(4-trans-propylcyclohexyl)-2-cyano-ethan.

1-(4-Propyl-1-bicyclo-(2,2,2)-octyl)-2-(4-trans-propylcyclohexyl)-1-fluoro-ethan
1-(4-Propyl-1-bicyclo-(2,2,2)-octyl)-2-(4-trans-propylcyclohexyl)-1-chloro-ethan
1-(4-Propyl-1-bicyclo-(2,2,2)-octyl)-2-(4-trans-propylcyclohexyl)-1-bromo-ethan
1-(4-Propyl-1-bicyclo-(2,2,2)-octyl)-2-(4-trans-propylcyclohexyl)-1-cyano-ethan

1-(4-Propyl-1-bicyclo-(2,2,2)-octyl)-2-(4-trans-propyloxycarbonylcyclohexyl)-1-fluoro-ethan
1-(4-Propyl-1-bicyclo-(2,2,2)-octyl)-2-(4-trans-propyloxycarbonylcyclohexyl)-1-cyano-ethan

1-(4-trans-Propylcyclohexyl)-2-(4-pentylphenyl)-1-fluoro-ethan
1-(4-trans-Propylcyclohexyl)-2-(4-pentylphenyl)-1-chloro-ethan
1-(4-trans-Propylcyclohexyl)-2-(4-pentylphenyl)-1-bromo-ethan
1-(4-trans-Propylcyclohexyl)-2-(4-pentylphenyl)-1-cyano-ethan
1-(4-trans-Propylcyclohexyl)-2-(4-butoxyphenyl)-1-fluoro-ethan
1-(4-trans-Propylcyclohexyl)-2-(4-butoxyphenyl)-1-chloro-ethan
1-(4-trans-Propylcyclohexyl)-2-(4-butoxyphenyl)-1-bromo-ethan
1-(4-trans-Propylcyclohexyl)-2-(4-butoxyphenyl)-1-cyano-ethan

1-(4-trans-Propylcyclohexyl)-2-(4-(4-trans-pentylcyclohexyl)-phenyl)-1-fluoro-ethan
1-(4-trans-Propylcyclohexyl)-2-(4-(4-trans-pentylcyclohexyl)-phenyl)-1-chloro-ethan
1-(4-trans-Propylcyclohexyl)-2-(4-(4-trans-pentylcyclohexyl)-phenyl)-1-bromo-ethan
1-(4-trans-Propylcyclohexyl)-2-(4-(4-trans-pentylcyclohexyl)-phenyl)-1-cyano-ethan

1-(4-trans-Propylcyclohexyl)-2-(4-(4-trans-pentylcyclohex-1-yl-methoxy)-phenyl)-1-fluoro-ethan
1-(4-trans-Propylcyclohexyl)-2-(4-(4-trans-pentylcyclohex-1-yl-methoxy)-phenyl)-1-chloro-ethan
1-(4-trans-Propylcyclohexyl)-2-(4-(4-trans-pentylcyclohex-1-yl-methoxy)-phenyl)-1-bromo-ethan
1-(4-trans-Propylcyclohexyl)-2-(4-(4-trans-pentylcyclohex-1-yl-methoxy)-phenyl)-1-cyano-ethan

1-(4-trans-Propylcyclohexyl)-2-(4-trans-(4-pentylphenyl)-cyclohexyl)-1-fluoro-ethan
1-(4-trans-Propylcyclohexyl)-2-(4-trans-(4-pentylphenyl)-cyclohexyl)-1-chloro-ethan
1-(4-trans-Propylcyclohexyl)-2-(4-trans-(4-pentylphenyl)-cyclohexyl)-1-bromo-ethan
1-(4-trans-Propylcyclohexyl)-2-(4-trans-(4-pentylphenyl)-cyclohexyl)-1-cyano-ethan
1-(4-trans-Propylcyclohexyl)-2-(4-trans-(4-butoxyphenyl)-cyclohexyl)-1-fluoro-ethan
1-(4-trans-Propylcyclohexyl)-2-(4-trans-(4-butoxyphenyl)-cyclohexyl)-1-chloro-ethan
1-(4-trans-Propylcyclohexyl)-2-(4-trans-(4-butoxyphenyl)-cyclohexyl)-1-bromo-ethan
1-(4-trans-Propylcyclohexyl)-2-(4-trans-(4-butoxyphenyl)-cyclohexyl)-1-cyano-ethan

1-(4-trans-Propylcyclohexyl)-2-(4-trans-(4-pentylphenoxymethyl)-cyclohexyl)-1-fluoro-ethan
1-(4-trans-Propylcyclohexyl)-2-(4-trans-(4-pentylphenoxymethyl)-cyclohexyl)-1-chloro-ethan
1-(4-trans-Propylcyclohexyl)-2-(4-trans-(4-pentylphenoxymethyl)-cyclohexyl)-1-bromo-ethan
1-(4-trans-Propylcyclohexyl)-2-(4-trans-(4-pentylphenoxymethyl)-cyclohexyl)-1-cyano-ethan

1-(4-trans-Propylcyclohexyl)-2-(4-trans-(4-butoxyphenoxymethyl)-cyclohexyl)-1-fluoro-ethan
1-(4-trans-Propylcyclohexyl)-2-(4-trans-(4-butoxyphenoxymethyl)-cyclohexyl)-1-chloro-ethan
1-(4-trans-Propylcyclohexyl)-2-(4-trans-(4-butoxyphenoxymethyl)-cyclohexyl)-1-bromo-ethan
1-(4-trans-Propylcyclohexyl)-2-(4-trans-(4-butoxyphenoxymethyl)-cyclohexyl)-1-cyano-ethan

1-(4-Propyl-1-bicyclo-(2,2,2)-octyl)-2-(4-trans-(4-pentylphenyl)-cyclohexyl)-1-fluoro-ethan
1-(4-Propyl-1-bicyclo-(2,2,2)-octyl)-2-(4-trans-(4-pentylphenyl)-cyclohexyl)-1-chloro-ethan
1-(4-Propyl-1-bicyclo-(2,2,2)-octyl)-2-(4-trans-(4-pentylphenyl)-cyclohexyl)-1-bromo-ethan
1-(4-Propyl-1-bicyclo-(2,2,2)-octyl)-2-(4-trans-(4-pentylphenyl)-cyclohexyl)-1-cyano-ethan

1-(4-Propyl-1-bicyclo-(2,2,2)-octyl)-2-(4-trans-(4-butoxyphenyl)-cyclohexyl)-1-fluoro-ethan
1-(4-Propyl-1-bicyclo-(2,2,2)-octyl)-2-(4-trans-(4-butoxyphenyl)-cyclohexyl)-1-chloro-ethan
1-(4-Propyl-1-bicyclo-(2,2,2)-octyl)-2-(4-trans-(4-butoxyphenyl)-cyclohexyl)-1-bromo-ethan
1-(4-Propyl-1-bicyclo-(2,2,2)-octyl)-2-(4-trans-(4-butoxyphenyl)-cyclohexyl)-1-cyano-ethan

1-(4-Propyl-1-bicyclo-(2,2,2)-octyl)-2-(4-(4-trans-pentylcyclohexyl)-phenyl-1-fluoro-ethan
1-(4-Propyl-1-bicyclo-(2,2,2)-octyl)-2-(4-(4-trans-pentylcyclohexyl)-phenyl-1-chloro-ethan
1-(4-Propyl-1-bicyclo-(2,2,2)-octyl)-2-(4-(4-trans-pentylcyclohexyl)-phenyl-1-bromo-ethan
1-(4-Propyl-1-bicyclo-(2,2,2)-octyl)-2-(4-(4-trans-pentylcyclohexyl)-phenyl-1-cyano-ethan

1-(4-Propyl-1-bicyclo-(2,2,2)-octyl)-2-(4-(4-trans-propyloxycarbonylcyclohexyl)-phenyl)-1-fluoro-ethan
1-(4-Propyl-1-bicyclo-(2,2,2)-octyl)-2-(4-(4-trans-propyloxycarbonylcyclohexyl)-phenyl)-1-chloro-ethan
1-(4-Propyl-1-bicyclo-(2,2,2)-octyl)-2-(4-(4-trans-propyloxycarbonylcyclohexyl)-phenyl)-1-bromo-ethan
1-(4-Propyl-1-bicyclo-(2,2,2)-octyl)-2-(4-(4-trans-propyloxycarbonylcyclohexyl)-phenyl)-1-cyano-ethan

1-(4-trans-Propyl-cyclohexyl)-2-(6-pentylpyridazin-3-yl)-1-fluoro-ethan
1-(4-trans-Propyl-cyclohexyl)-2-(6-pentylpyridazin-3-yl)-1-chloro-ethan
1-(4-trans-Propyl-cyclohexyl)-2-(6-pentylpyridazin-3-yl)-1-bromo-ethan
1-(4-trans-Propyl-cyclohexyl)-2-(6-pentylpyridazin-3-yl)-1-cyano-ethan

1-(4-trans-Propylcyclohexyl)-2-(6-butoxypyridazin-3-yl)-1-fluoro-ethan
1-(4-trans-Propylcyclohexyl)-2-(6-butoxypyridazin-3-yl)-1-chloro-ethan
1-(4-trans-Propylcyclohexyl)-2-(6-butoxypyridazin-3-yl)-1-bromo-ethan
1-(4-trans-Propylcyclohexyl)-2-(6-butoxypyridazin-3-yl)-1-cyano-ethan

1-(4-trans-Propylcyclohexyl)-2-(4-trans-(6-pentylpyridazin-3-yl)-cyclohexyl)-1-fluoro-ethan
1-(4-trans-Propylcyclohexyl)-2-(4-trans-(6-pentylpyridazin-3-yl)-cyclohexyl)-1-chloro-ethan
1-(4-trans-Propylcyclohexyl)-2-(4-trans-(6-pentylpyridazin-3-yl)-cyclohexyl)-1-bromo-ethan
1-(4-trans-Propylcyclohexyl)-2-(4-trans-(6-pentylpyridazin-3-yl)-cyclohexyl)-1-cyano-ethan

1-(4-trans-Propylcyclohexyl)-2-(4-trans-(6-butoxypyridazin-3-yl)-cyclohexyl)-1-fluoro-ethan
1-(4-trans-Propylcyclohexyl)-2-(4-trans-(6-butoxypyridazin-3-yl)-cyclohexyl)-1-chloro-ethan
1-(4-trans-Propylcyclohexyl)-2-(4-trans-(6-butoxypyridazin-3-yl)-cyclohexyl)-1-bromo-ethan
1-(4-trans-Propylcyclohexyl)-2-(4-trans-(6-butoxypyridazin-3-yl)-cyclohexyl)-1-cyano-ethan
1-(4-trans-Propylcyclohexyl)-2-(4-trans-(2-(6-pentylpyridazin-3-yl)-ethyl)-cyclohexyl)-1-fluoro-ethan
1-(4-trans-Propylcyclohexyl)-2-(4-trans-(2-(6-pentylpyridazin-3-yl)-ethyl)-cyclohexyl)-1-chloro-ethan
1-(4-trans-Propylcyclohexyl)-2-(4-trans-(2-(6-pentylpyridazin-3-yl)-ethyl)-cyclohexyl)-1-bromo-ethan
1-(4-trans-Propylcyclohexyl)-2-(4-trans-(2-(6-pentylpyridazin-3-yl)-ethyl)-cyclohexyl)-1-cyano-ethan

1-(4-trans-Propylcyclohexyl)-2-(4-trans-(2-(6-butoxypyridazin-3-yl)-ethyl)-cyclohexyl)-1-fluoro-ethan
1-(4-trans-Propylcyclohexyl)-2-(4-trans-(2-(6-butoxypyridazin-3-yl)-ethyl)-cyclohexyl)-1-bromo-ethan
1-(4-trans-Propylcyclohexyl)-2-(4-trans-(2-(6-butoxypyridazin-3-yl)-ethyl)-cyclohexyl)-1-chloro-ethan
1-(4-trans-Propylcyclohexyl)-2-(4-trans-(2-(6-butoxypyridazin-3-yl)-ethyl)-cyclohexyl)-1-cyano-ethan

1-(4-trans-Propylcyclohexyl)-2-(6-(4-trans-pentylcyclohexyl)-pyridazin-3-yl)-1-fluoro-ethan
1-(4-trans-Propylcyclohexyl)-2-(6-(4-trans-pentylcyclohexyl)-pyridazin-3-yl)-1-chloro-ethan
1-(4-trans-Propylcyclohexyl)-2-(6-(4-trans-pentylcyclohexyl)-pyridazin-3-yl)-1-bromo-ethan
1-(4-trans-Propylcyclohexyl)-2-(6-(4-trans-pentylcyclohexyl)-pyridazin-3-yl)-1-cyano-ethan

1-(4-trans-Propylcyclohexyl)-2-(6-(2-(4-trans-pentylcyclohexyl)-ethyl)-pyridazin-3-yl)-1-fluoro-ethan
1-(4-trans-Propylcyclohexyl)-2-(6-(2-(4-trans-pentylcyclohexyl)-ethyl)-pyridazin-3-yl)-1-chloro-ethan
1-(4-trans-Propylcyclohexyl)-2-(6-(2-(4-trans-pentylcyclohexyl)-ethyl)-pyridazin-3-yl)-1-bromo-ethan
1-(4-trans-Propylcyclohexyl)-2-(6-(2-(4-trans-pentylcyclohexyl)-ethyl)-pyridazin-3-yl)-1-cyano-ethan

1,2-Bis-(4-trans-cyanomethylcyclohexyl)-1-fluoro-ethan
1,2-Bis-(4-trans-cyanopropylcyclohexyl)-1-fluoro-ethan

1-(4'-trans-Fluoropropyl-4-trans-bicyclohexyl)-2-(4-trans-fluoropropylcyclohexyl)-2-chloro-ethan
1-(4'-trans-Fluoropropyl-4-trans-bicyclohexyl)-2-(4-trans-fluoropropylcyclohexyl)-2-cyano-ethan
1-(4'-trans-Fluoropropyl-4-trans-bicyclohexyl)-2-(4-trans-fluoropropyloxycarbonylcyclohexyl)-2-chloro-ethan
1-(4'-trans-Fluoropropyl-4-trans-bicyclohexyl)-2-(4-trans-fluoropropyloxycarbonylcyclohexyl)-2-cyano-ethan

1-(4-trans-(2-(4-trans-Cyanopropylcyclohexyl)-ethyl)-cyclohexyl)-2-(4-trans-propylcyclohexyl)-2-fluoro-ethan
1-(4-trans-(2-(4-trans-Fluoropropylcyclohexyl)-ethyl)-cyclohexyl)-2-(4-trans-propylcyclohexyl)-2-fluoro-ethan
1-(4-trans-(2-(4-trans-Chloropropylcyclohexyl)-ethyl)-cyclohexyl)-2-(4-trans-propylcyclohexyl)-2-chloro-ethan
1-(4-trans-(2-(4-trans-Cyanopropylcyclohexyl)-ethyl)-cyclohexyl)-2-(4-trans-propylcyclohexyl)-2-cyano-ethan
1-(4-trans-Cyanopropylcyclohexyl)-2-(4-pentylphenyl)-1-fluoro-ethan
1-(4-trans-Fluoropropylcyclohexyl)-2-(4-pentylphenyl)-1-fluoro-ethan
1-(4-trans-Chloropropylcyclohexyl)-2-(4-pentylphenyl)-1-chloro-ethan
1-(4-trans-Cyanopropylcyclohexyl)-2-(4-pentylphenyl)-1-cyano-ethan
1-(4-trans-Cyanopropylcyclohexyl)-2-(4-butoxyphenyl)-1-fluoro-ethan
1-(4-trans-Fluoropropylcyclohexyl)-2-(4-butoxyphenyl)-1-fluoro-ethan

1,2-Bis-(4-trans-heptylcyclohexyl)-1-cyanoethan
1,2-Bis-(4-trans-hexylcyclohexyl)-1-cyanoethan
1,2-Bis-(4-trans-butylcyclohexyl)-1-cyanoethan
1,2-Bis-(4-trans-propylcyclohexyl)-1-cyanoethan
1-(4-trans-propylcyclohexyl)-2-(4-trans-butylcyclohexyl)-1-cyanoethan
1-(4-trans-propylcyclohexyl)-2-(4-trans-pentylcyclohexyl)-1-cyanoethan
1-(4-trans-propylcyclohexyl)-2-(4-trans-hexylcyclohexyl)-1-cyanoethan
1-(4-trans-propylcyclohexyl)-2-(4-trans-heptylcyclohexyl)-1-cyanoethan
1-(4-trans-butylcyclohexyl)-2-(4-trans-pentylcyclohexyl)-1-cyanoethan
1-(4-trans-butylcyclohexyl)-2-(4-trans-hexylcyclohexyl)-1-cyanoethan
1-(4-trans-butylcyclohexyl)-2-(4-trans-heptylcyclohexyl)-1-cyanoethan
1-(4-trans-pentylcyclohexyl)-2-(4-trans-hexylcyclohexyl)-1-cyanoethan
1-(4-trans-pentylcyclohexyl)-2-(4-trans-heptylcyclohexyl)-1-cyanoethan
1-(4-trans-hexylcyclohexyl)-2-(4-trans-heptylcyclohexyl)-1-cyanoethan.

Es folgen Beispiele für erfindungsgemäße Flüssigkristallmischungen mit mindestens einer Komponente der Formel (1) :

**Beispiel A**

Eine Flüssigkristallmischung aus

11 Gew.-% trans,trans-4-Propyl-4'-methoxycyclohexylcyclohexan
10 Gew.-% trans,trans-4-Propyl-4'-ethoxycyclohexylcyclohexan,
4 Gew.-% trans,trans-4-Propylcyclohexylcyclohexan-4'-carbonsäure-trans-4-propylcyclohexylester,
4 Gew.-%trans,trans-4-Propylcyclohexylcyclohexan-4'-carbonsäure-trans-4-pentylcyclohexylester,
4 Gew.-% trans,trans-4-Butylcyclohexylcyclohexan-4'-carbonsäure-trans-4-propylcyclohexylester,
4 Gew.-% trans,trans-4-Butylcyclohexylcyclohexan-4'-carbonsäure-trans-4-pentylcyclohexylester,
34 Gew.-% 1-(4-trans-Butylcyclohexyl)-2-(4-transheptylcyclohexyl)-1-cyanoethan und
29 Gew.-% 1,2-Bis-(4-trans-Pentylcyclohexyl)-1-cyanoethan
weist eine negative DKA auf.

**Beispiel B**

Eine Flüssigkristallmischung aus
20 Gew.-% 1,2-Bis-(4-trans-Propylcyclohexyl)-1-cyanoethan,
21 Gew.-% 1,2-Bis-(4-trans-Pentylciclohexyl)-1-cyanoethan,
22 Gew.-% 1-(4'-trans-Pentyl-4-trans-bicyclohexyl)-2-(4-trans-pentylcyclohexyl)-2-cyanoethan,
11 Gew.-% trans,trans-4-Propyl-4'-methoxycyclohexylcyclohexan
10 Gew.-% trans,trans-4-Propyl-4'-ethoxycyclohexylcyclohexan,
4 Gew.-% trans,trans-4-Propylcyclohexylcyclohexan-4'-carbonsäure-trans-4-propylcyclohexylester,
4 Gew.-% trans,trans-4-Propylcyclohexylcyclohexan-4'-carbonsäure-trans-4-pentylcyclohexylester,
4 Gew.-% trans,trans-4-Butylcyclohexylcyclohexan-4'-carbonsäure-trans-4-propylcyclohexylester,
4 Gew.-% trans,trans-4-Butylcyclohexylcyclohexan-4'-carbonsäure-trans-4-pentylcyclohexylester,
weist eine negative DKA auf.

**Beispiel C**

Eine Flüssigkristallmischung aus
30 Gew.-% 1-(4-trans-Butylcyclohexyl)-2-(4-transheptylcyclohexyl)-1-cyanoethan
16 Gew.-% trans,trans-4-Propyl-4'-methoxycyclohexylcyclohexan
15 Gew.-% trans,trans-4-Propyl-4'-ethoxycyclohexylcyclohexan,
4 Gew.-% trans,trans-4-Propylcyclohexylcyclohexan-4'-carbonsäure-trans-4-propylcyclohexylester,
4 Gew.-% trans,trans-4-Propylcyclohexylcyclohexan-4'-carbonaäure-trans-4-pentylcyclohexylester,
4 Gew.-% trans,trans-4-Butylcyclohexylcyclohexan-4'-carbonsäure-trans-4-propylcyclohexylester,
4 Gew.-% trans,trans-4-Butylcyclohexylcyclohexan-4'-carbonsäure-trans-4-pentylcyclohexylester,
8 Gew.-% trans-4-Propylcyclohexancarbonsäure-(trans-4-propylcyclohexylester),
7 Gew.-% trans-4-Pentylcyclohexancarbonsäure-(trans-4-pentylcyclohexylester) und
8 Gew.-% trans-4-Hexylcyclohexancarbonsäure-(trans-4-heptylcyclohexylester)
weist eine negative DKA auf.

**Patentansprüche**

1. Anisotrope Verbindungen, die mindestens einen querpolarisierenden Substituenten tragen, dadurch gekennzeichnet, dass die Verbindungen der Formel

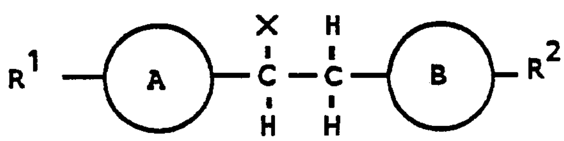

(1)

entsprechen, in der X eine Cyanogruppe oder ein Halogenatom ist und der Ring A gewählt ist aus cycloaliphatischen Resten der Formeln (1a) oder (1b)

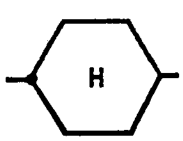

(1a)

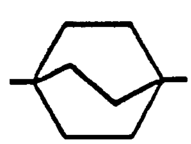

(1b)

und der Ring B gewählt ist aus den cycloaliphatischen Resten der Formeln (1a) und (1b) oder aus aromatischen Resten der Formeln (1c) oder (1d)

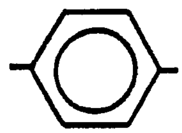

(1c)

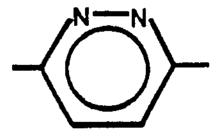

(1d)

und $R^1$ und $R^2$ gleiche oder verschiedene Reste der Formel (1e)

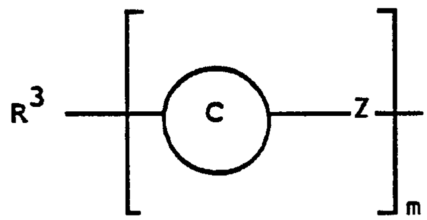

(1e)

bedeuten, in welcher der Ring C die für Ring B angegebene Bedeutung hat und $R^3$ gewählt ist aus Wasserstoff und Alkyl-, Alkoxy-, Alkoxycarbonyl- oder Alkylcarbonyloxygruppen, deren Alkylteile jeweils 1-12 C-Atome in gerader oder verzweigter und gegebenenfalls chiraler Kohlenstoffkette enthalten und die gegebenenfalls einen oder mehrere Substituenten gewählt and Halogen und Cyano tregen, wobei an jeweils einem Atom der Kohlenstoffkette höchstens einer der genannten Substituenten hängt, Z die Kovalenzbindung oder eine Brückengruppe der Formeln $-CH_2O$, $-C(X^1)(H)-CH_2$ oder -COO bedeutet, wobei $X^1$ Wasserstoff ist oder die für X angegebene Bedeutung hat, und m jeweils 0 oder 1 bedeutet, mit den Massgaben, dass

(a) an keinen im Molekül vorhandenen aromatischen Rest der Formeln (1c) oder (1d) Gruppen der Formeln $-CH_2O$ oder -C(X)(H)- direkt mit den C-Atomen dieser Gruppen gebunden sind ausser wenn X=F, und

(b) $R^3$ nicht Wasserstoff ist, wenn m 0 bedeutet

oder der Ring C ein cyclischer Rest der Formel (1a) oder (1b) ist.

2. Verbindung nach Anspruch 1, dadurch gekennzeichnet, dass X die Cyanogruppe ist.

3. Verbindung nach Anspruch 1, dadurch gekennzeichnet, dass X Fluor, Chlor oder Brom ist.

4. Verbindung nach einem der Ansprüche 1-3, dadurch gekennzeichnet, dass das Molekül der Formel (1) insgesamt zwei oder drei cycloaliphatische Reste der Formeln (1a) oder (1b) enthält.

5. Verbindung nach einem der Ansprüche 1-4, dadurch gekennzeichnet, dass das Molekül der Formel (1) höchstens eine Carboxylgruppe enthält.

6. Verbindung nach einem der Ansprüche 1-5, dadurch gekennzeichnet, dass der Alkylteil von $R^3$ 3-9 C-Atome enthält.

7. Verbindung nach einem der Ansprüche 1-6, dadurch gekennzeichnet, dass der Alkylteil von $R^3$ höchstens zwei der genannten Substituenten trägt.

8. Verbindung nach Anspruch 1, gekennzeichnet durch die Formel (2)

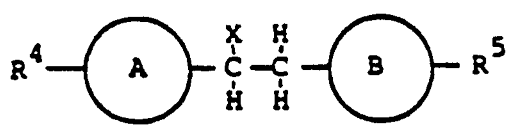

(2)

in der X, A und B die in Anspruch 1 angegebene Bedeutung haben und $R^4$, $R^5$ gleich oder verschieden sind und die für $R^3$ angegebene Bedeutung mit Ausnahme von Wasserstoff haben.

9. Verbindung nach Anspruch 1, gekennzeichnet durch die Formel (3)

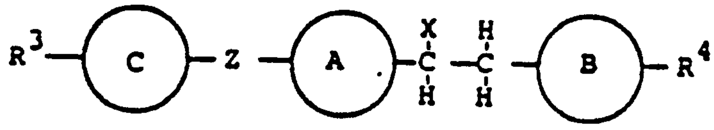

(3)

in der X, A, B, C, Z und $R^3$ die in Anspruch 1 angegebene Bedeutung haben und $R^4$ die für $R^3$ angegebene Bedeutung hat.

10. Verbindung nach Anspruch 1, gekennzeichnet durch die Formel (4)

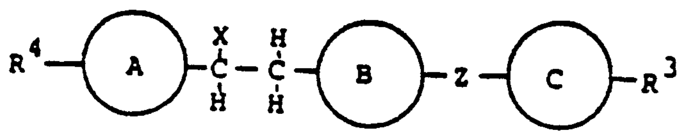

(4)

in der X, A, B, C, Z und $R^3$ die in Anspruch 1 angegebene Bedeutung haben und $R^4$ die für $R^3$ angegebene Bedeutung hat.

11. Verbindung nach einem der Ansprüche 8-10, dadurch gekennzeichnet, dass X die Cyanogruppe oder Fluor oder Chlor ist.

12. Verbindung nach Anspruch 1, gekennzeichnet durch die Formel (5)

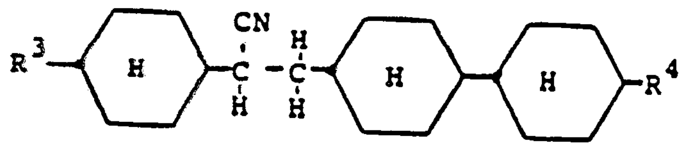

(5)

in der $R^4$ die in Anspruch 1 für $R^3$ angegebene Bedeutung hat und $R^3$, $R^4$ gleich oder verechieden sind aber nicht Wasserstoff bedeuten.

13. Verbindung nach Anspruch 12, dadurch gekennzeichnet, dass $R^3$ und $R^4$ jeweils einen $C_1$-$C_{12}$-Alkylrest, z. B. n-Pentylrest, bedeuten.

14. Verbindung nach einem der Ansprüche 1-13, dadurch gekennzeichnet, dass die Alkylteile von $R^3$ einen oder höchstens zwei Substituenten tragen und diese an C-Atomen der Alkylkette hängen, welche vom zugehörigen Ring durch nicht mehr als 2 Atome getrennt sind.

15. Flüssigkristallmischung, dadurch gekennzeichnet, dass sie mindestens eine anisotrope Verbindung gemäss einem der Ansprüche 1-14 enthält.

16. Elektrooptisches Anzeigeelement, dadurch gekennzeichnet, das es eine Flüssigkristallmischung nach Anspruch 15 enthält.

17. Verwendung einer Verbindung gemäß einem der Ansprüche 1 - 14 als Komponente einer Flüssigkristallmischung.

**Claims**

1. Anisotropic compounds which carry at least one cross-polarising substituent characterised in that the compounds correspond to the formula

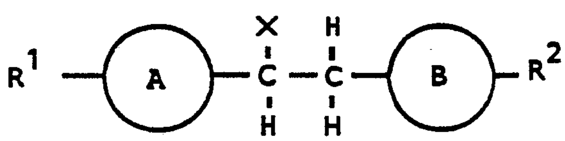

(1)

in which X is a cyano group or a halogen atom, the ring A is chosen from the cycloaliphatic radicals of the formula (1a) or (1b)

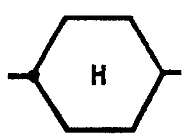

(1a)

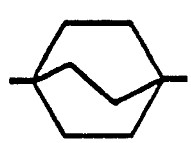

(1b)

the ring B is chosen from the cycloaliphatic radicals of the formulae (1a) and (1b) or from the aromatic radicals of the formula (1c) or (1d)

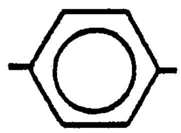

(1c)

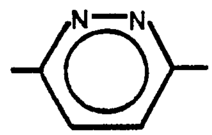

(1d)

and $R^1$ and $R^2$ are identical or different radicals of the formula (1e)

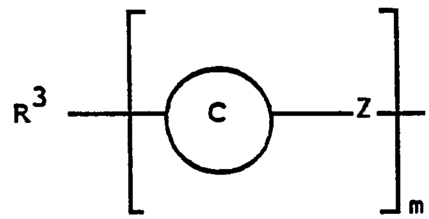

(1e)

in which the ring C has the meaning given for ring B, $R^3$ is chosen from hydrogen and alkyl, alkoxy, alkoxy-carbonyl and alkylcarbonyloxy groups, the alkyl parts of which each contain 1-12 C atoms in a straight or branched, optionally chiral carbon chain and which optionally carry one or more substituents taken from halogen and cyano, at most one of the substituents mentioned in each case being attached to one atom of the carbon chain, Z is the covalent bond or a bridge group of the formula $-CH_2O-$, $-C(X^1)(H)-CH_2-$ or -COO-, wherein $X^1$ is hydrogen or has the meaning given for X, and m is in each case 0 or 1, with the provisos that

(a) groups of the formula $-CH_2O-$ or -C(X)(H)- are not bonded directly via their C atoms to any aromatic radical of the formula (1c) or (1d) present in the molecule, except for if X=F, and

(b) $R^3$ is not hydrogen if m is 0 or if the ring C is a cyclic radical of the formula (1a) or (1b).

2. Compound according to Claim 1, characterised in that X is the cyano group.

3. Compound according to Claim 1, characterised in that X is fluorine, chlorine or bromine.

4. Compound according to one of Claims 1-3, characterised in that the molecule of the formula (1) contains a total of two or three cycloaliphatic radicals of the formula (1a) or (1b).

5. Compound according to one of Claims 1-4, characterised in that the molecule of the formula (1) contains at most one carboxyl group.

6. Compound according to one of Claims 1-5, characterised in that the alkyl part of $R^3$ contains 3-9 C atoms.

7. Compound according to one of Claims 1-6, characterised in that the alkyl part of $R^3$ carries at most two of the substituents mentioned.

8. Compound according to Claim 1, characterised by the formula (2)

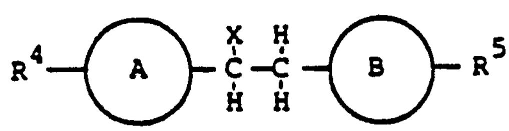

(2)

in which X, A and B have the meaning given in Claim 1 and $R^4$ and $R^5$ are identical or different and have the meaning given for $R^3$, with the exception of hydrogen.

9. Compound according to Claim 1, characterised by the formula (3)

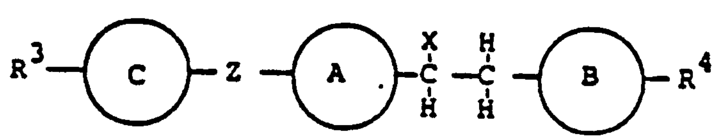

(3)

in which X, A, B, C, Z and $R^3$ have the meaning given in Claim 1 and $R^4$ has the meaning given for $R^3$.

10. Compound according to Claim 1, characterised by the formula (4)

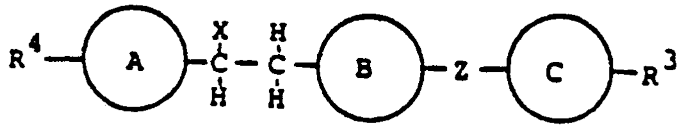

(4)

in which X, A, B, C, Z and $R^3$ have the meaning given in Claim 1 and $R^4$ has the meaning given for $R^3$.

11. Compound according to one of Claims 8-10, characterised in that X is the cyano group or fluorine or chlorine.

12. Compound according to Claim 1, characterised by the formula (5)

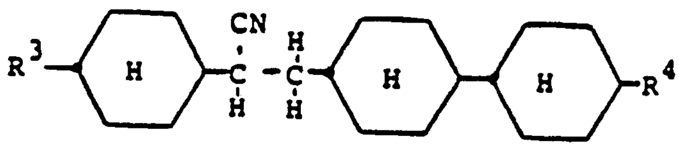

(5)

in which $R^4$ has the meaning given for $R^3$ in Claim 1 and $R^3$ and $R^4$ are identical or different but are not hydrogen.

13. Compound according to Claim 12, characterised in that $R^3$ and $R^4$ are each a $C_1$-$C_{12}$-alkyl radical, for example an n-pentyl radical.

14. Compound according to one of Claims 1-13, characterised in that the alkyl parts of $R^3$ carry one or at most two substituents and these are attached to C atoms of the alkyl chain which are separated from the associated ring by not more than 2 atoms.

15. Liquid crystal mixture, characterised in that it contains at least one anisotropic compound according to one of Claims 1-14.

16. Electrooptical display element, characterised in that it contains a liquid crystal mixture according to Claim 15.

17. Use of a compound according to one of the Claims 1 - 14 as component of a liquid crystal mixture.

**Revendications**

1. Composés anisotropes portant au moins un substituant provoquant la polarisation transversale, caractérisés en ce qu'ils répondent à la formule (1)

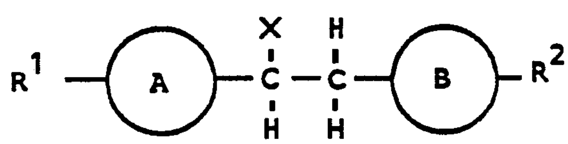

(1)

dans laquelle X représente un groupe cyano ou un atome d'halogène et le noyau A est choisi parmi les radicaux cycloaliphatiques de formule (1a) ou (1b)

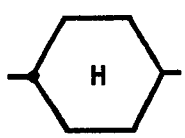

(1a)

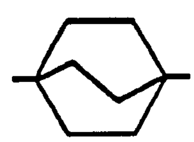

(1b)

et le cycle B est choisi parmi les radicaux cycloaliphatiques de formules (1a) et (1b) ou parmi les radicaux aromatiques de formule (1c) ou (1d)

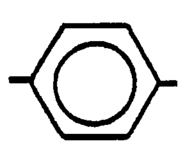

(1c)

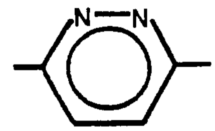

(1d)

et $R^1$ et $R^2$ représentent des radicaux identiques ou différents de formule (1e)

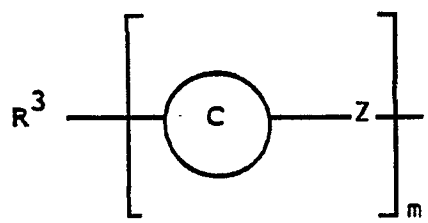

(1e)

dans laquelle le cycle C a les significations indiquées pour le cycle B et $R^3$ est choisi parmi l'hydrogène et les groupes alkyle, alcoxy, alcoxycarbonyle ou alkylcarbonyloxy dont les parties alkyle contiennent chacune 1 à 12 atomes de carbone dans une chaîne carbonée droite ou ramifiée et, le cas échéant, chirale, et qui porte éventuellement un ou plusieurs substituants choisis parmi les halogènes et les groupes cyano, chaque atome de la chaîne carbonée portant au maximum un seul des substituants mentionnés, Z représente une liaison covalente ou un groupe de pont de formule $-CH_2O-$, $-C(X^1)(H)-CH_2-$ ou -COO-, $X^1$ représente l'hydrogène ou a l'une des significations indiquées pour X, et m est égal à 0 ou 1 avec les restrictions suivantes :

(a) les groupes de formules $-CH_2O-$ ou -C(X)(H)-ne peuvent être reliés directement par leurs atomes de carbone à un radical aromatique de formule (1c) ou (1d) présent dans la molécule, sauf lorsque X=F, et

(b) $R^3$ ne peut représenter l'hydrogène lorsque m est égal d à ou que le radical C est un radical cyclique de formule (1a) ou (1b).

2. Composé selon la revendication 1, caractérisé en ce que X représente le groupe cyano.

3. Composé selon la revendication 1, caractérisé en ce que X représente le fluor, le chlore ou le brome.

4. Composé selon l'une des revendications 1 à 3, caractérisé en ce que la molécule de formule (1) contient au total 2 ou 3 radicaux cycloaliphatiques de formules (1a) ou (1b).

5. Composé selon l'une des revendications 1 à 4, caractérisé en ce que la molécule de formule (1) contient au maximum un groupe carboxyle.

6. Composé selon l'une des revendications 1 à 5, caractérisé en ce que la partie alkyle de $R^3$ contient 3 à 9 atomes de carbone.

7. Composé selon l'une des revendications 1 à 6, caractérisé en ce que la partie alkyle de $R^3$ porte au maximum deux des substituants mentionnés.

8. Composé selon la revendication 1, caractérisé par la formule (2)

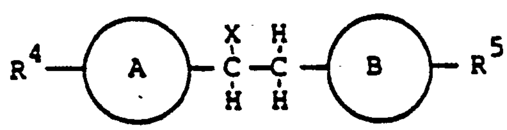

(2)

dans laquelle X, A et B ont les significations indiquées dans la revendication 1 et $R^4$ et $R^5$, ayant des significations identiques ou différentes, ont les significations indiquées pour $R^3$ à l'exception de l'hydrogène.

9. Composé selon la revendication 1, caractérisé par la formule (3)

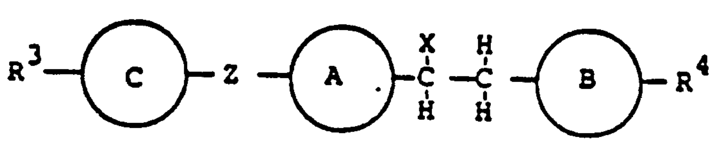

(3)

dans laquelle X, A, B, C, Z et $R^3$ ont les significations indiquées dans la revendication 1 et $R^4$ les significations indiquées pour $R^3$.

10. Composé selon la revendication 1, caractérisé par la formule (4)

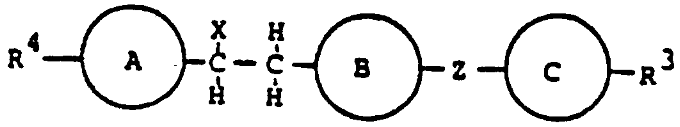

(4)

dans laquelle X, A, B, C, Z et $R^3$ ont les significations indiquées dans la revendication 1 et $R^4$ les significations indiquées pour $R^3$.

11. Composé selon l'une des revendications 8 à 10, caractérisé en ce que X représente le groupe cyano ou le fluor ou le chlore.

12. Composé selon la revendication 1, caractérisé par la formule (5)

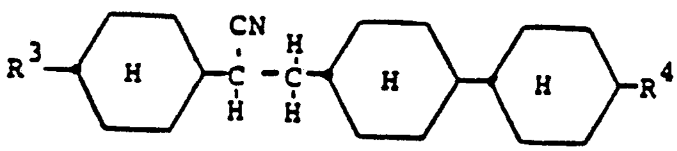

(5)

dans laquelle $R^4$ a les significations indiquées pour $R^3$ dans la revendication 1, et $R^3$ et $R^4$ ont des significations identiques ou différentes mais ne peuvent représenter l'hydrogène.

13. Composé selon la revendication 12, caractérisé en ce que $R^3$ et $R^4$ représentent chacun un groupe alkyle en C1-C12, par exemple un groupe n-pentyle.

14. Composé selon l'une des revendications 1 à 13, caractérisé en ce que les parties alkyle de $R^3$ portent un ou au maximum deux substituants qui sont placés sur des atomes de carbone de la chaîne alkyle séparé par deux atomes au maximum du cycle voisin.

15. Mélange à cristaux liquides caractérisé en ce qu'il contient au moins un composé anisotrope selon l'une des revendications 1 à 14.

16. Elément d'affichage électro-optique caractérisé en ce qu'il contient un mélange à cristaux liquides selon la revendication 15.

17. Utilisation d'un composé selon l'une des revendications 1 à 14 en tant que composant d'un mélange à cristaux liquides.